# EUROPEAN PATENT APPLICATION

(11) **EP 4 230 135 A1**
(43) Date of publication of application: **23.08.2023**
(21) Application number: 21789874.1
(22) Date of filing: 13.10.2021
(51) Int. Cl.: A61B 5/153, A61B 34/30, A61B 5/15

(54) **UNIT FOR COLLECTING BLOOD AND/OR PLASMA**

(30) Priority: 15.10.2020 EP 20382902
(71) Applicant: Grifols Worldwide Operations Limited, Dublin 22 (IE)
(72) Inventor: BOIRA BONHORA, Jordi, 08150 Parets del Valles (Barcelona) (ES); ROURA SALIETTI, Carlos, 08150 Parets del Valles (Barcelona) (ES)
(74) Representative: Durán-Corretjer, S.L.P.
(86) International application number: PCT/IB2021/059395
(87) International publication number: WO 2022/079628

(57) **Abstract**

Unit for automatically collecting blood and/or plasma, comprising a plurality of stations for automatically collecting blood and/or plasma and at least one venepuncture robot. The robot has means for detecting blood vessels. Said robot is fitted on a linear guide and is movable on said linear guide. The stations are laid out along the linear guide such that the robot can move along the linear guide to access said stations.

## Description

The present application relates to a unit for collecting blood and/or plasma.

More specifically, the present invention discloses a unit for collecting blood and/or plasma that is advantageous compared with known units.

In blood and/or plasma donation centres, it is common to perform a vein-puncturing process (also known as venepuncture) on the person, donor or a patient from whom blood and/or plasma is to be collected. During this process, the user's vein from which the collection will be performed is located, selected and established in order to then make a puncture and perform said collection. Normally, this process is done manually, with the member of the medical staff being the person who deals with the patient and performs the entire process.

In addition, the person responsible for performing said collection often has trouble establishing the ideal vein to perform the collection. This results in a lack of precision when establishing the site at which to make the puncture, which may cause a poor puncture in those donors for whom it is difficult to locate the veins, posing a risk for both the donor and the nurse.

Currently, systems comprising means for automatically detecting blood vessels are known, these systems usually being robots. Said systems locate the blood vessels in the patient by means of an infrared camera, which emits a ray of near-infrared light (NIR), and by means of ultrasound. The blood of the patients captures the emitted light, thereby making it possible to detect the position of the veins. Next, the vessels are reconstructed in 2D or 3D using image analysis. The system can then take that image and depict it on the skin of the patient, thereby simplifying the work for the medical staff, allowing the puncture to be made in the best possible area, improving convenience for the donor and the waiting time, and reducing the possibilities of having to make more than one puncture when the vein cannot be found on the first try.

Recently, robots have been disclosed that, in addition to automatically detecting the vein in the patient, also automatically perform venepuncture, the robot itself making the puncture and collecting the blood and/or plasma. In this respect, automatic systems for collecting blood from a user are known, as are methods for placing an intravenous catheter in the vein of a user and collecting blood from said vein. In these cases, the robot can locate the blood vessels of the patient by means of near-infrared light (NIR) and ultrasound, and can insert the needle directly into the middle of a vein without any intervention from medical staff. Once the blood has been collected, the medical staff take care of the retrieval of the needle.

Normally, these robots can be laid out in a fixed manner at each one of the stations, or can be easy to transport by being movable on the ground, or can be light enough to be moved by the medical staff. In this respect, robots for automatic venepuncture that are small or medium-sized, or those having supports that can move said robots manually from one patient to another, are known. The Chinese utility model document CN209074628 U discloses an automatic chair for collecting blood, while US patent document US8888714 B discloses robots positioned on detachable supports having wheels. These systems are useful as support for nurses, but they are inefficient when it comes to quickly collecting blood and/or plasma from several patients.

The donation centres may be large and have many stations for patients, said stations being beds, gurneys, chairs or other types of stations; alternatively, the centres may be small and have only a few stations. In both cases, the use of said venepuncture robots (both the robots that automatically detect blood vessels and those that comprise puncturing means and blood-collecting means) is advantageous, but their layout in said centres may be inconvenient.

One disadvantage of the donation centres and blood-collecting units having fixed robots is that they require the presence of a robot at each one of the stations, or they have a limited number of stations having a robot, in which case it is not possible to have a robot available for a patient until a previous patient has finished their procedure with that robot, meaning that patients have to wait to be seen and the capacity for collection from several patients is simultaneously low. This low capacity makes these layouts slow and inefficient, with high amounts of lost time and low cost-efficiency. Moreover, a disadvantage of the donation centres having movable robots is that they require staff to move the robots constantly, whether manually or by controlling them remotely, which entails high staff costs. In addition, the presence of movable robots moving around continuously is inconvenient for patients and for the medical staff themselves, since they constitute obstacles to movement within the donation centre. Another disadvantage of the current layouts is that, in order to see a high number of people or patients, a large-scale layout of stations is required so that the robots can move freely around them.

Therefore, there is a need for a collection unit that is adapted to centres for donating and collecting blood and/or plasma, comprises said venepuncture robots and allows automatic venepuncture to be performed safely on several patients, as well as for a donation centre layout that makes it possible to deal with a high number of patients quickly and safely.

An object of the present invention is thus to disclose a unit that overcomes the above-described disadvantages, simplifying venepuncture in donors for whom it is difficult to locate veins, increasing the precision of said venepuncture and allowing venepuncture to be performed safely.

It is also an object of the present invention to disclose a system for collecting blood and/or plasma that takes up little space and can be implemented efficiently in any type of donation centre, even those that are small.

More specifically, the present invention discloses a unit for automatically collecting blood and/or plasma, comprising a plurality of stations for automatically collecting blood and/or plasma and at least one robot, said robot having means for detecting blood vessels, having the special feature whereby said robot is fitted on a linear guide and is movable on said linear guide, and whereby the stations are laid out along the linear guide such that the robot can move along the linear guide to access said stations.

This layout of stations (i.e. of places where patients, donors or individuals are dealt with) along a linear guide allows just one robot to access several stations quickly and efficiently, without having to move along the ground, which causes a nuisance among patients, thereby ensuring an efficient layout of the space since the same space can include a greater number of stations. Preferably, said stations are gurneys.

Preferably, the robot is an articulated robot comprising rotary means and/or joints. Said rotary means and/or joints allow the robot to access a greater number of stations as well as a greater number of positions in the same station.

Preferably, the stations are laid out on either side of the linear guide. More preferably, the stations are laid out on either side of the linear guide in a staggered manner. Alternatively, the stations are laid out uniformly and symmetrically on either side of the linear guide.

Preferably, the means for detecting blood vessels comprise an ultrasound machine. Preferably, the means for detecting blood vessels comprise an infrared camera. More preferably, the means for detecting blood vessels comprise an ultrasound machine and an infrared camera.

Preferably, the robot comprises puncturing means. More preferably, the robot comprises means for puncturing the tissue of a patient. Even more preferably, the puncturing means comprise a needle, a clamp for supporting said needle, and a linear actuator. Even more preferably, the robot comprises a needle corrector.

Alternatively, the robot can detect blood vessels in such a way that the tissue of a patient is punctured by a person, more preferably by medical staff.

Preferably, the unit comprises at least one signal for indicating the presence of patients at the stations. More preferably, the unit comprises one signal at each of the stations for indicating the presence of patients at said stations.

Preferably, the linear guide comprises rails. These rails simplify the linear movement of the robots across the entire guide. Preferably, the linear guide has means for being mounted on the ground. Alternatively, the linear guide has means for being mounted on the ceiling of a facility. In that case, the robot can access the stations by moving along the linear guide from a higher position.

Preferably, the unit comprises more than one robot.

Additionally, the present invention also discloses a multi-patient layout of stations, comprising a robot on a linear guide.

Additionally, the present invention also discloses a blood and/or plasma donation centre, having the special feature whereby it comprises at least one unit for automatically collecting blood and/or plasma according to that described above.

To aid understanding, drawings showing an example embodiment of the present invention are included by way of an explanatory yet non-limiting example.
Fig. 1 is a diagram of an example embodiment of a unit for automatically collecting blood and/or plasma according to the present invention.
Fig. 2 is an enlarged view of part of the diagram from Fig. 1.
Fig. 3 is a schematic perspective view of the example embodiment from Fig. 1 and 2.
Fig. 4 shows a detail of the robot of the example embodiment from the previous figures.

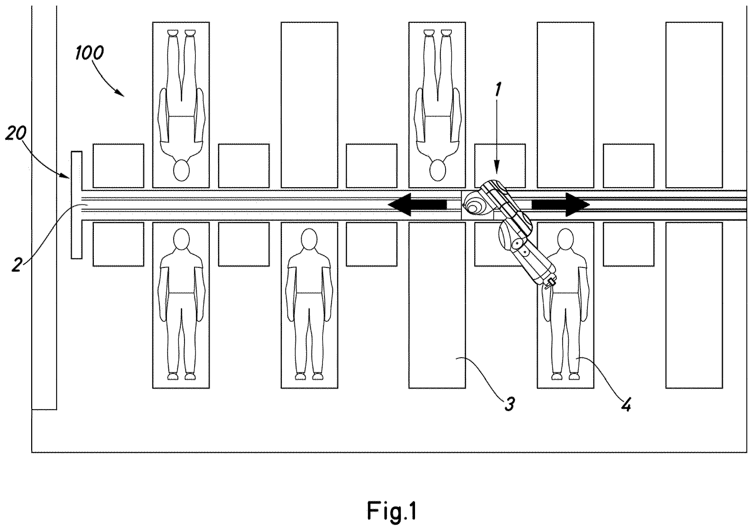

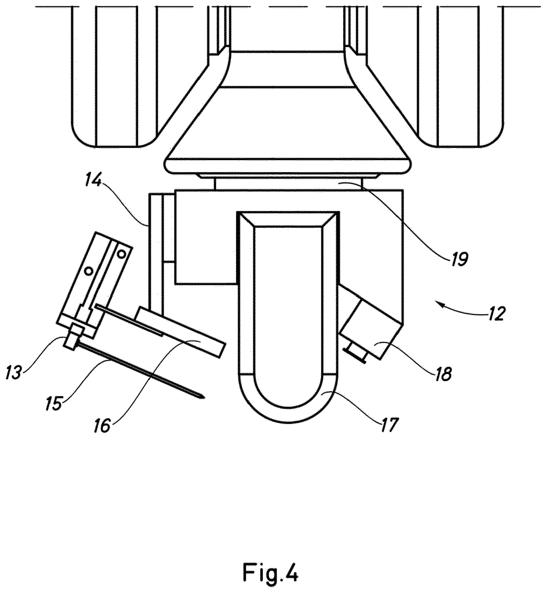

Fig. 1 to 4 disclose an example embodiment of a unit for automatically collecting blood and/or plasma according to the present invention.

Fig. 1 shows a unit 100 for automatically collecting blood and/or plasma according to an embodiment of the present invention. Said unit 100 comprises a plurality of stations 3 for automatically collecting blood and/or plasma and at least one venepuncture robot 1, said robot 1 being fitted on a linear guide 2 and the robot 1 being movable on said linear guide 2.

As shown in Fig. 1, the stations 3 are laid out along the linear guide 2 such that the robot 1 can move along the linear guide 2 to access said stations 3. Stations should be understood as being the places or locations where a patient, donor or individual is located; they can be beds, gurneys, chairs or simply a place designated for that purpose, and their specific features are not part of the present invention. The patient 4 or donor is positioned in said station to wait to be seen by the robot 1.

These stations 3 are laid out such that the robot 1 can access them from the linear guide 2. More specifically, the robot 1 can access the patients 4 positioned at the stations 3 such that it can interact with them, performing an ultrasound, detecting blood vessels, collecting blood, collecting plasma, etc. In Fig. 1, the stations 3 are shown laid out in a uniform and symmetrical manner along the linear guide. Alternatively, the stations 3 can be mounted in a staggered manner along the linear guide 2, while other non-described station layouts are also possible.

In Fig. 1, the linear guide 2 is shown positioned on a structure 20, in a central hub between stations. This structure 20 can be anchored to the ground, as shown in Fig. 1, or, alternatively, can be positioned on the ceiling of a facility. Fig. 1 also shows that the linear guide 2 comprises rails. These rails simplify the movement of the robot 1 along the guide 2 such that the robot can access the stations 3 laid out along said guide. The direction of movement of the robot 1 is shown in Fig. 1 by means of arrows.

This layout is advantageous compared with current layouts in which the robot is a robot that has movable elements and can move between stations, since with this layout there are fewer elements that obstruct movement of people within the donation centre. Another advantage of the present invention is that this unit 100 is laid out such as to take up less space within a storey, building or facility than other layouts in which the robot is not positioned on a linear guide, thus making it ideal to be implemented as a unit or cell for collecting blood and/or plasma in places with little space. Due to its compact size, making it possible to use a large range of spaces and facilities as blood and/or plasma donation centres.

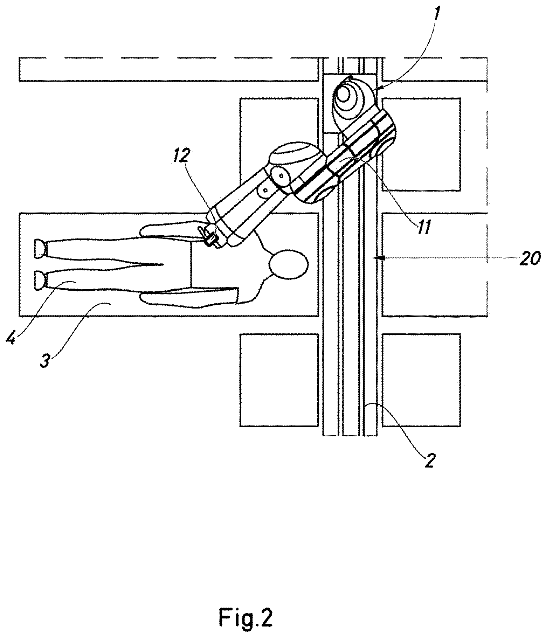

Fig. 2 to 4 show the venepuncture robot of the embodiment from Fig. 1 in more detail.

The robot 1 comprises means for detecting blood vessels and means for performing venepuncture on a patient 4, said blood vessel detection means and venepuncture means being of the known type. In the example shown in the drawings, said means are positioned at a distal end 12 of an arm 11 of the robot 1. The means for detecting blood vessels comprise an infrared camera 18, or a camera with light in the NIR spectrum, and an ultrasound machine 17. The means for performing the venepuncture comprise a needle 15. This needle can be held in a clamp 13 and controlled by means of linear actuators 14, 16. Other configurations different from those shown are also possible.

It should be noted that the area on which the detection and/or puncturing is performed is any tissue of the body of the patient, and that this patient can be any kind of person, whether a patient, a sick person, a donor or a participant in a scientific study. Besides humans, this unit may also be usable for collecting blood and/or plasma from animals.

The robot 1 of the present invention accesses a station 3 by moving along the linear guide 2 and locates the blood vessels of a patient 4 by means of the camera 18 and by means of ultrasound 17. The light emitted by the camera 18 is captured by the blood of the patient, allowing the robot 1 to detect the position of the veins. Next, the robot 1 reconstructs the vessels in 2D or 3D using image analysis. The robot 1 then makes the puncture on the basis of the results of the ultrasound, using said ultrasound as a guide for placing the needle 15. The patient then rests with the needle inserted and the blood and/or plasma is collected. This process can be performed by the robot itself or by the medical staff, thereby freeing up the robot to access a new station. Once the collection is complete, the needle is removed from the patient by the medical staff, while the robot 1 is supplied with a new needle. The robot 1 can also have means (not shown) for taking a new needle automatically.

In an alternative, not preferred configuration, the robot may not have puncturing means, with the medical staff being responsible for making the puncture. In this configuration, the medical staff use the reconstruction of the vessels in 2D or 3D, performed by the robot using image analysis, as an aid to make the puncture at the most suitable point on the patient. Alternatively, the robot depicts the results of the ultrasound on the patient by means of a projector (not shown in the drawings), thereby further simplifying the puncturing process.

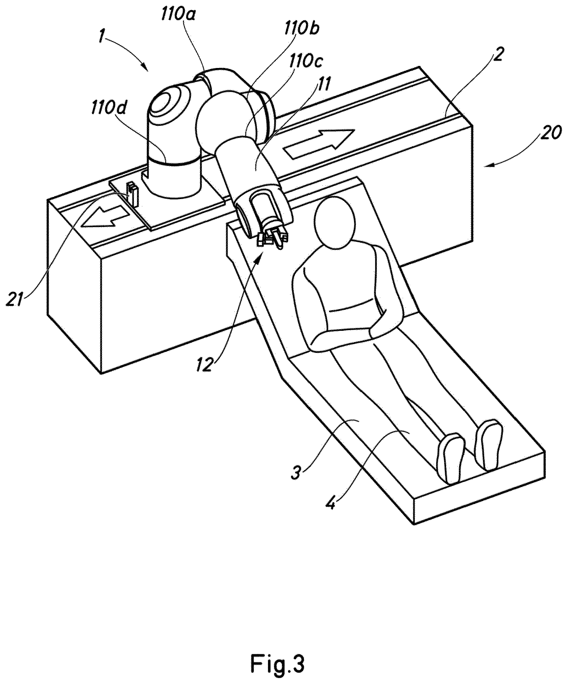

The robot 1 also comprises a rotary element 19, which enables the correct arrangement of the robot for detecting the vessels or making the puncture, thereby improving the precision thereof. In addition, besides the linear actuators 14, 16 for correctly placing the needle 15, the robot can also comprise a needle corrector 21 for aligning the position of the needle with respect to the robot arm (shown in Fig. 3 at the base of the robot 1). The robot 1 in the drawings also comprises several joints 110a, 110b, 110c, 110d on its arm 11 or on its body, which improve the range of motion of the robot and help it access the stations 3.

The unit 100 may also comprise a signal (not shown) that indicates the presence of patients at each of the stations 3. This signal is a known status signal associated with each of the stations and is received by the robot. On the basis of this signal, the robot can automatically establish which station to move towards and then access. One example of such a signal would be a signal having three output statuses: empty station, station with waiting patient, station with treated patient. Additionally, the layout comprises a light signal such that the medical staff can also know the status of each one of the patients. This light signal can be shown in each of the stations 3 or together on a monitor.

In another embodiment (not shown), the unit for collecting blood and/or plasma comprises more than one robot, the unit being able to comprise robots having means for detecting blood and/or plasma, robots having venepuncture means and robots having both means.

Other configurations, having different robots used during the venepuncture process, e.g. robots for placing an intravenous catheter in the vein of a user and collecting blood from that vein, are also possible in a collection unit according to the present invention.

The present invention also discloses a station layout along a linear guide positioned on a structure, as shown in Fig. 1.

The present invention also discloses a blood and/or plasma donation centre, characterised in that it comprises at least one unit for automatically collecting blood and/or plasma according to that described above.

Although the invention has been described and illustrated with reference to several representative examples, it should be understood that those example embodiments do not limit the present invention in any way, and that any of the variants covered by the content of the attached claims, whether directly or by way of equivalence, should be considered to fall under the scope of the present invention.

## Claims

1. Unit for automatically collecting blood and/or plasma, comprising a plurality of stations for automatically collecting blood and/or plasma and at least one robot, said robot having means for detecting blood vessels, **characterised in that** said robot is fitted on a linear guide and is movable on said linear guide, and **in that** the stations are laid out along the linear guide such that the robot can move along the linear guide to access said stations.

2. Unit according to claim 1, **characterised in that** the robot is an articulated robot comprising rotary means and/or joints.

3. Unit according to any one of the preceding claims, **characterised in that** the stations are laid out on either side of the linear guide.

4. Unit according to any one of the preceding claims, **characterised in that** the means for detecting blood vessels comprise an ultrasound machine and an infrared camera.

5. Unit according to any one of the preceding claims, **characterised in that** the robot comprises means for puncturing the tissue of a patient.

6. Unit according to claim 5, **characterised in that** the puncturing means comprise a needle, a clamp for supporting said needle, and a linear actuator.

7. Unit according to any one of the preceding claims, **characterised in that** it comprises at least one signal for indicating the presence of patients at the stations.

8. Unit according to any one of the preceding claims, **characterised in that** the guide comprises rails.

9. Unit according to any one of the preceding claims, **characterised in that** it comprises more than one robot.

10. Blood and/or plasma donation centre, **characterised in that** it comprises at least one unit for automatically collecting blood and/or plasma according to any one of claims 1 to 9.
